# EUROPEAN PATENT APPLICATION

(11) **EP 4 806 507 A1**
(43) Date of publication of application: **16.09.2026**
(21) Application number: 25163145.3
(22) Date of filing: 12.03.2025
(51) Int. Cl.: A61N 7/00, A61H 9/00, A61H 23/00, A61H 23/02

(54) **BLOOD PRESSURE LOWERING DEVICE**

(30) Priority: 10.03.2025 LT 2025508
(71) Applicant: Kaunas University of Technology, 44249 Kaunas (LT)
(72) Inventor: Ostasevicius, Vytautas, 54359 Domeikava, Kaunas (LT); Bubulis, Algimantas, 52177 Kaunas (LT); Jurenas, Vytautas, Kaunas (LT)
(74) Representative: AAA Law

(57) **Abstract**

According to one aspect of the invention, the invention is a blood pressure lowering device comprising two identical piezoelectric transducers (P, N) for attachment to the surface of the human body (1), near the chest and on the back, in the lung area, each of which comprises an adhesive sealing tape (2), an elastic film (3), a piezoelectric film (4) covering a perforated soft layer (5), a pressed vacuum bag (6), an air extraction nozzle (7), electrodes (8), and air removal openings (9).

According to another aspect of the invention, the invention is a method of using a blood pressure lowering and blood gas exchange accelerating device comprising extraction of air from a vacuum bag (6) through an air extraction nozzle (7), where a force is created in the vacuum bag (6) to press a piezoelectric film (4) with electrodes (8) against the human body (1) in the lung area, where the piezoelectric film (4) thus pressed is activated in the frontal and the rear piezoelectric transducers (P, N) of the blood pressure lowering and blood gas exchange accelerating device.

## Description

### Technical Field

The invention is attributed to the medical field and can be used to improve the physiological parameters of blood.

### Technical Level

When blood is exposed to low-frequency ultrasound, which emits travelling acoustic waves, erythrocyte aggregates dissociate into single erythrocytes, whose concentration per unit of blood volume decreases due to the space between them, and the hemoglobin contained in them participates in gas exchange over the entire surface of the single erythrocyte [1]. A decrease in the number of single erythrocytes in the blood volume reduces the blood viscosity and, at the same time, reduces the blood pressure and the pulse compared to the number of aggregated erythrocytes in the same blood volume unaffected by ultrasound. The ability of hemoglobin in aggregated erythrocytes to participate in gas exchange across the entire erythrocyte surface is limited due to the erythrocyte surfaces sticking together. In medical practice, high-frequency (0.5 - 30 MHz) ultrasound emitting a standing acoustic wave is widely used for diagnostics and therapy. Until now, the focus has been on the effect of ultrasound on the endothelium of vascular walls [2] causing obstructive lesions of the main arteries, which usually result in acute coronary or peripheral vascular syndromes. It has been found that the effect of low-frequency ultrasound, which emits a travelling acoustic wave, on various blood components is stronger than that of high-frequency ultrasound. Erythrocytes are particularly sensitive [3] to this, which can be disrupted by higher levels of ultrasound intensity, thus causing hemolysis of the blood. In order to therapeutically affect internal organs, such as the heart, liver, kidneys or lungs, and their blood circulation with acoustic waves, low-frequency ultrasound is more effective than high-frequency, because it creates a more hydrodynamic than a thermal effect in biological tissues, and due to lower attenuation in biological tissues, the travelling waves of ultrasound have a bigger effect on internal organs. However, during the dissociation of erythrocyte aggregates, it is important not to exceed ultrasound intensity level that causes hemolysis of the blood.

There is a known therapeutic method that uses a low-frequency ultrasound transducer. The transducer is designed to treat chronic wounds and, to the author's knowledge, is the first portable device that shortens wound healing time while reducing healthcare costs. The transducer emits ultrasound in the low frequency range (from 20 to 100 kHz), the intensity level of which does not cause temperature effects and cavitation in biological tissues [4]. To achieve this goal, patent No. EP1339366 - "Vacuum therapy for a cleaned wound" is known.

There is a known European patent No. EP4374840 "Vacuum therapy apparatus with ultrasonic stimulation and method of controlling such apparatus", in which the therapeutic effect of low-frequency ultrasound on biological tissues is created by exciting ultrasonic frequency vibrations in a vacuum cup with the help of piezoceramic rings, the vibrations of which are controlled by a controller synchronously with a vacuum pump.

There is also known European patent application No. EP 23168014.1 "Leg blood flow stimulation system and method for combining low and high frequency acoustic vibration", according to which speakers placed in a chamber excite low frequency acoustic vibrations acting on the lower legs, and high frequency vibrations in the soles of the feet are created by piezoelectric bimorph-type transducers, the operation of which is synchronized with the low frequency vibrations emitted by the speakers.

The technical solutions mentioned cannot be fully applied to the stimulation of pulmonary blood flow because they create a local focusing ultrasound field, the therapeutic effect of which will cover only a small part of the pulmonary blood flow.

### Short Description of the Invention

The invention is a blood pressure lowering device and method of using such device. The blood pressure lowering device comprises two low-frequency ultrasound transducers, which comprise a large-area, thin (less than 0.2 mm thick) and flexible piezoelectric film, hermetically connected by its contour to a vacuum bag, the edges of which have an adhesive tape attached, with the help of which the piezoelectric film with the vacuum bag is hermetically attached to the human body, in the area of lungs. In such transducers, the piezoelectric film is tightly pressed against the human body with the help of a vacuum bag generated and emits low-intensity and low-frequency ultrasonic waves over a large area of the lungs, under the influence of which erythrocyte aggregates dissociate into single erythrocytes, whose hemoglobin participates in gas exchange across the entire surface of the erythrocyte. The number of single erythrocytes separated from aggregates by low-frequency ultrasound decreases in the blood volume due to the gaps between them, which reduces the blood viscosity and at the same time reduces the blood pressure and the pulse compared to the case where in the same blood volume a larger number of erythrocytes are not aggregated by ultrasound. Hemoglobin molecules, of which are up to 300 million in each erythrocyte, come into contact with oxygen across single erythrocyte entire surface when they reach the lungs in the bloodstream. This significantly accelerates blood gas exchange. The ability of hemoglobin in aggregated erythrocytes to participate in gas exchange across the entire erythrocyte surface is limited due to the erythrocyte surfaces sticking together.

### Short Description of Drawings

The invention is explained in the drawings. The accompanying diagrams and drawings form an integral part of the invention description and are given as a reference to a possible embodiment of the invention but are not intended to limit the scope of the invention.
Fig. 1 shows a diagram of the application of the device for lowering blood pressure and accelerating blood gas exchange.
Fig. 2 shows a diagram of the device for lowering blood pressure and accelerating blood gas exchange.

### Detailed description of the invention

The device for lowering blood pressure and accelerating blood gas exchange according to one embodiment of the invention comprises two identical piezoelectric front transducers (P) and rear transducers (N) on the human body surface (1), in the lung area, on the chest and on the back, and an elastic and electrically insulating film (3) connecting the said transducers to each other from their sides. Each of the aforementioned transducers comprise an adhesive sealing tape (2) for sealing the interaction area between the transducers and the human body surface (1), a flexible, elastic and electrically insulating film (3) for connecting and attaching the piezoelectric transducers (P, N) to the human body during use, a piezoelectric film (4) for creating vibrations, covering a perforated soft layer (5), a pressable vacuum bag (6), a nozzle for applying vacuum to the pressable vacuum bag (7), and electrodes (8) for applying electric current to the piezoelectric film.

An elastic and electrically insulating film (3) is attached to the adhesive sealing tape (2) for attaching the transducers (P, N) to the human body along its entire contour, where a piezoelectric film (4) with electrodes (8) is attached to the adhesive sealing tape (2) along its entire area, while the electrodes are connected to an ultrasonic generator (not shown in the drawing). The area of the piezoelectric film (4) corresponds to the area of the adhesive sealing film (2). The piezoelectric film (4) is covered with a perforated soft layer (5) having mounted the vacuum bag (6) for controlling the pressing force of the vacuum bag (6), preferably rectangular in shape, but which may also constitute another shape, is hermetically connected along its entire contour to the contour of the adhesive sealing tape (2) and the contour of the piezoelectric film (4) and the contour of the insulating film (3) which is larger than the contour of the adhesive sealing tape (2). A vacuum is created in the vacuum bag (6) by extracting air through the nozzle (7).

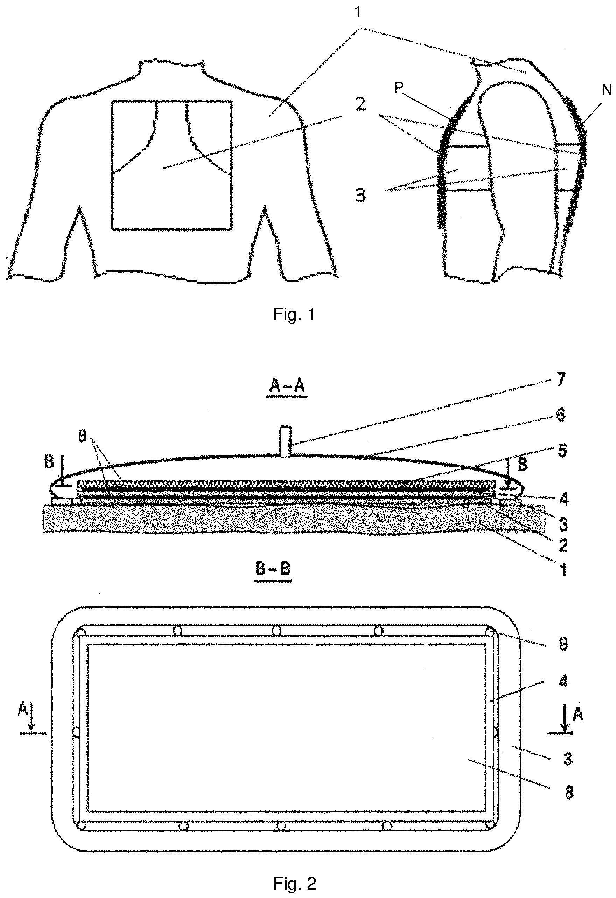

The method of using the device for reducing blood pressure and accelerating blood gas exchange comprises the following steps: with the help of an adhesive sealing tape (2) and an insulating film (3), the transducers are attached to the human body over their entire surface in the lung area on the chest and on the back if necessary; air is extracted from the vacuum bag (6) through the air extraction nozzle (7) and a vacuum is created, thus creating a force for pressing the piezoelectric film (4) with electrodes (8) against the surface of the human body (1) in the lung area over the entire surface area of the transducers, in the chest (P) and back (N) elements of the device for reducing blood pressure and accelerating blood gas exchange (Fig. 1). Thus, at the minimum power of ultrasonic wave generation by the piezoelectric film (4), in the entire area of effect on the body, both in the front part (P) and in the back part (N), it is possible to transmit low-frequency ultrasound vibrations from two sides to the lungs, with minimal impact on other human organs. In addition, the adhesive sealing tape (2) has openings (9) at opposite edges of its contour, through which air is removed from the interaction area between the insulating film (3) and the surface of the human body at the time when the vacuum is created in the vacuum bag (6).

### Citation sources for reference

1. Mayu Hatakeyama, Yuichiro Akamatsu, Takayuki Sato " Investigation of formation and ultrasonic disassembling of RBC Aggregation aimed for maintenance of blood channel" - Hatakevama - 2022 - Electronics and Communications in Japan - Wilev Online Library. 2022Wilev Periodicals LLC. p.1 -9.
2. Agile Tunaityte, Silvijus Abramavicius, Augusta Volkeviciute, Mantas Venslauskas, Algimantas Bubulis, Vytis Bajoriunas, Ulf Simonsen , Vytautas Ostaśevicius, Vytautas Jurenas. Kasparas Briedis, Edgaras Stankevicius. "Contractions Induced in Human Pulmonary Arteries by a H2S Donor, GYY 4137, are Inhibited by Low-Frequency (20 kHz) Ultrasound", Journal Biomolecules 2024, 14a 257s 2
3. US Patent No. US6450979.
4. Olivia Ngo, Ewan Niemann, Vivinya Gunasekaran, Prabagar Sankar, Miriam Putterman. Alec Lafontant, Sumati Nadkami, Rose Ann DiMaria-Ghalili, Michael Neidrauer, Leonid, IEEE. "Transactions on ultrasonics, ferroelectrics, and frequency control", vol. 66, no. 3, March 2019.

## Claims

1. A blood pressure lowering device comprising piezoelectric transducers, **characterized in that** it comprises two identical piezoelectric transducers (P, N) for attachment to the surface of the human body, in the lung area on the chest and on the back, each of which comprises an adhesive sealing tape (2), a flexible and elastic insulating film (3) connecting said identical transducers (P, N), a piezoelectric film (4), a perforated soft layer (5) covering the piezoelectric film (4), a vacuum bag (6) pressing the sealing tape (2), the piezoelectric film (4) and the perforated soft layer (5) covering the human body when the blood pressure lowering device is used, a nozzle (7) for creating a vacuum in the vacuum bag (6), electrodes (8) of the piezoelectric film (4), air removal openings (9) for removing air from the interaction area between the human body and the aforementioned identical piezoelectric transducers (P, N).

2. Blood pressure lowering device according to the first claim, wherein an insulating film (3) is attached to the adhesive sealing tape (2) along its entire contour, on which a piezoelectric film (4) with electrodes (8) is attached along its entire area, which are connected to an ultrasonic generator, wherein the piezoelectric film (4) is covered with a perforated soft layer (5) on which a pressed vacuum bag (6) is attached.

3. A method of using a device for reducing blood pressure, comprising activation of piezoelectric elements, **characterized in that** a vacuum is created in a vacuum bag (6) through an air extraction nozzle (7), where the vacuum bag (6) creates a force for pressing a piezoelectric film (4) with electrodes (8) against the human body (1) in the lung area, air is removed through the air removal openings (9) from the interaction area between the insulating film (3) and the human body, the pressed piezoelectric film (4) is activated in the frontal and the rear piezoelectric transducers (P, N) of the device for reducing blood pressure and accelerating blood gas exchange.
